# FASCICULE DE BREVET EUROPEEN

(11) **EP 4 119 134 B1**
(45) Date de publication et mention de la délivrance du brevet: **19.11.2025**
(21) Numéro de dépôt: 22315076.4
(22) Date de dépôt: 25.03.2022
(51) Int. Cl.: A61K 31/015, A61K 31/045, A61K 31/11, A61K 31/12, A61K 31/353, A61K 31/575, A61P 3/06, A61P 3/10, A61P 31/12, A61P 31/14, A61P 31/16, A61P 31/18, A61P 35/00

(54) **COMPOSITION PHARMACEUTIQUE DESTINÉE À INHIBER L'INFECTIOSITÉ DES VIRUS À BICOUCHE LIPIDIQUE, À TRAITER LES MALADIES ASSOCIÉES ET LEURS COMPLICATIONS**
PHARMAZEUTISCHE ZUSAMMENSETZUNG ZUR HEMMUNG DER INFEKTIOSITÄT VON LIPID-DOPPELSCHICHTVIREN, ZUR BEHANDLUNG VON DAMIT VERBUNDENEN KRANKHEITEN UND DEREN KOMPLIKATIONEN
PHARMACEUTICAL COMPOSITION FOR USE IN INHIBITING THE INFECTIVITY OF BILAYER VIRUSES, TO TREAT ASSOCIATED DISEASES AND THEIR COMPLICATIONS

(43) Date de publication de la demande: 18.01.2023
(73) Titulaire: Monkam Nitcheu, Guy Faustin, 97232 Le Lamentin (MQ)
(72) Inventeur: Monkam Nitcheu, Guy Faustin, 97232 Le Lamentin (MQ)
(74) Mandataire: Garavelli, Paolo

(56) Documents cités:
- EP-A1- 3 785 709
- WO-A1-2012/130893
- WO-A1-2018/127748
- JP-A- H0 826 980
- ROSHDY WAEL H. ET AL: "EGYVIR: An immunomodulatory herbal extract with potent antiviral activity against SARS-CoV-2", PLOS ONE, vol. 15, no. 11, 18 November 2020 (2020-11-18), pages e0241739, XP055846128, DOI: 10.1371/journal.pone.0241739
- BHATTACHARYA RIYA ET AL: "Antiviral activity of bioactive phytocompounds against coronavirus: An update", JOURNAL OF VIROLOGICAL METHODS, ELSEVIER BV, NL, vol. 290, 23 January 2021 (2021-01-23), XP086520479, ISSN: 0166-0934, [retrieved on 20210123], DOI: 10.1016/J.JVIROMET.2021.114070
- RAJAN MARIAPPAN ET AL: "Promising Antiviral Molecules from Ayurvedic Herbs and Spices against COVID-19", CHINESE JOURNAL OF INTEGRATED MEDICINE /ZHONGGUO JIEHE YIXUE ZAZHI (ENGLISH), vol. 27, no. 4, 5 February 2021 (2021-02-05), pages 243 - 244, XP037390321, ISSN: 1672-0415, DOI: 10.1007/S11655-021-3331-8
- Z�GOLO MAR�A ANTONELA ET AL: "Virtual screening of plant-derived compounds against SARS-CoV-2 viral proteins using computational tools", SCIENCE OF THE TOTAL ENVIRONMENT, ELSEVIER, AMSTERDAM, NL, vol. 781, 17 March 2021 (2021-03-17), XP086574866, ISSN: 0048-9697, [retrieved on 20210317], DOI: 10.1016/J.SCITOTENV.2021.146400
- KULKARNI SEEMA A ET AL: "Computational evaluation of major components from plant essential oils as potent inhibitors of SARS-CoV-2 spike protein", JOURNAL OF MOLECULAR STRUCTURE, ELSEVIER AMSTERDAM, NL, vol. 1221, 4 July 2020 (2020-07-04), XP086367096, ISSN: 0022-2860, [retrieved on 20200704], DOI: 10.1016/J.MOLSTRUC.2020.128823
- ASIF MUHAMMAD ET AL: "COVID-19 and therapy with essential oils having antiviral, anti-inflammatory, and immunomodulatory properties", INFLAMMOPHARMACOLOGY, vol. 28, no. 5, 2020, pages 1153 - 1161, XP037376410, ISSN: 0925-4692, DOI: 10.1007/S10787-020-00744-0
- CLERCQ DE E: "CURRENT LEAD NATURAL PRODUCTS FOR THE CHEMOTHERAPY OF HUMAN IMMUNODEFICIENCY VIRUS (HIV) INFECTION", MEDICINAL RESEARCH REVIEWS, WILEY SUBSCRIPTION SERVICES, INC., A WILEY COMPANY, US, vol. 20, no. 5, 1 September 2000 (2000-09-01), pages 323 - 349, XP009008036, ISSN: 0198-6325, DOI: 10.1002/1098-1128(200009)20:5<323::AID-MED1>3.0.CO;2-A

## Description

La présente invention concerne une composition pharmaceutique qui peut être utilisée comme anti-infectieux sur des agents pathogènes enveloppés, et notamment les coronavirus, et leurs différentes complications.

### Art Anterieur

La pandémie de Covid-19 est une pandémie d'une maladie infectieuse émergente, appelée Covid-19, provoquée par le coronavirus SARS-CoV-2. La pathogenèse de la Covid-19 est très hétérogènes et les personnes infectées ne développent pas forcément des symptomes. Les manifestations les plus fréquemment relevées dans la littérature bibliographique sont les signes classiques d'une infection respiratoire : fièvre, toux, gène respiratoire par accumulation de liquide dans les bronchioles. On peut y retrouver d'autres signes moins systématiques, variables selon l'âge, notamment les céphalées, malaises, chutes à répétition ou un état confusionnel, congestions nasales, maux de gorge, nausées, vomissements, diarrhées, perte de goût, perte d'odorat, érythème, éruption et plus rarement engelures.

Le traitement de la maladie consiste à en soulager les symptômes : Les antalgiques de type paracétamol et les antidiarrhéïques pour la fièvre et la diarrhées, le repos pendant toute la durée de l'infection, l'hydratation avec de l'eau pour éviter une déshydratation liée à la fièvre et des courbatures. D'autre part, l'oxygénothérapie, la prévention des thromboses, les anti-arythmiques, les anticoagulants ou encore la dialyse peuvent être mise en place en complément des traitements symptomatiques.

Par ailleurs de nombreux travaux ont été instauré afin de mieux comprendre ce virus et la maladie qu'il provoque, d'identifier de nouvelles cibles thérapeutiques. Cela a permis d'établir de nouvelles stratégies thérapeutiques :
- La vaccination : L'arrivée des vaccins contre le Covid-19 suscite un élan d'espoir dans la lutte contre l'infection à SARS-CoV-2. Quid de l'efficacité des vaccins sur le SARS-CoV-2 et ses variants à long terme.
- La plasmothérapie : Elle consiste à extraire le plasma des patients récupérés et à les donner à ceux qui sont gravement malade d'une manière similaire à un vaccin.
- L'immunothérapie : Le but est d'utiliser les médicaments à base d'anticorps pour prévenir l'hospitalisation des patients à risque de forme sévère de Covid-19, notamment chez ceux présentant des comorbidités telles que, les pathologies cardiovasculaires, les pathologies chroniques respiratoires, obésité, diabète, insuffisance rénale chronique, immunodépression liée au VIH ou au cancer. Cependant de nombreuses intérrogations sur l'immunogénécité, la spécificité, l'innocuité et le potentiel thérapeutique restent sans réponse.
Il n'existe aujourd'hui aucun traitement infectieux qui puisse guérir exclusivement la Covid-19. L'utilisation d'immunostimulants, d'antiviraux et d'antioxydants peut aider à réduire le risque de Covid-19.

Dans ce contexte infectieux, la membrane des cellules joue un rôle prépondérant dans un grand nombre de processus physiologiques ou physiopathologiques. En effet, elle comporte la plupart des éléments essentiels aux échanges entre la cellule et son environnement. Elle est constituée de micro-domaines encore appelés rafts. Ces derniers sont constitués d'un assemblage compacte de lipides (glycosphingolipides et/ou de la sphingomyéline et du cholestérol) et des protéines (protéines ancrées au glycosylphosphatidylinositol, des protéines liées au cholestérol, des protéines transmembranaires, des tyrosines kinases doublement acétylées de la famille Src, des sous-unités alpha des protéines G hétérotrimériques). Le cholestérol jouant un rôle de «colle» dynamique qui rigidifie, structure ces micro-domaines. Ces protéines, notamment les récepteurs ACE2, TMPRSS2 et bien d'autres encore ont un rôle déterminant dans la signalisation cellulaire, et sont impliquées dans certains processus inflammatoires, infectieux, d'installation des troubles du métabolisme et de cancérogenèse. D'autre part, le dysfonctionnement ou les modifications structurelles qui accompagnent l'activation de cette membrane par des agents pathogènes ou des signaux endogènes peuvent être à l'origine d'une surproduction de microparticules qui contribuent à amplifier l'inflammation, l'hyperactivation immunitaire systémique, la susceptibilité à la coagulation, l'initiation, la progression et l'aggravation de pathologies cardiovasculaires notamment, l'athérosclérose, l'infarctus du myocarde, l'accident vasculaire cérébral et le cancer.

L'inflammation virale expliquerait une bonne part importante des troubles du syndrome métabolique, notamment chez les sujets infectés par le SARS-CoV-2.

Par ailleurs, le d-limonene est connu pour son potentiel antiseptique, antiviral. Il possède aussi des propriétés antidiabétiques et hypolipémiantes, et peut à cet effet être considéré comme un agent potentiel pour prévenir et traiter les troubles métaboliques classiquement rencontrés dans les maladies infectieuses. Ses propriétés antioxydantes, anti-inflammatoires et anticancéreuses sont connues. Chez l'homme, le d-limonène a démontré une faible toxicité après une dose unique et répétée pendant un an.

Le beta-sitostérol et le lupéol sont des phytostérols ayant une structure moléculaire similaire à celle du cholestérol. A cet effet, ils peuvent entrer en compétition avec ce dernier. Leurs rôles dans les plantes sont identiques à celle du cholestérol chez l'homme, à savoir maintenir la structure et la fonction de la membrane cellulaire. De nombreuses données scientifiques ont montré que ces stérols possèdent des propriétés hypoglycémiantes, hypolipémiantes, anti-inflammatoires, antipyrétiques, immunostimulantes et anticancéreuses. Ils peuvent inhiber l'entrée du SARE-CoV-2 dans les cellules en limitant l'adhésion de la protéine Spike au récepteur ACE2. Leur potentiel pharmacologique en fait d'eux, des agents thérapeutiques dans les troubles du syndrome métabolique et les infections par agents pathogènes à bicouche lipidique.

Le cinnamaldéhyde est connu pour ses propriétés hypoglycémiantes, hypolipémiantes et anticancéreuses. Ses propriétés antibactériennes, antifongiques, immunostimulantes et antivirales sont connues.

Les documents de l'art antérieur les plus pertinents pour la présente invention sont:
D1 WO 2018/127748 A1 (MONKAM NITCHEU GUY FAUSTIN [FR]) 12 juillet 2018 (2018-07-12)
D2 EP 3 785 709 A1 (MONKAM NITCHEU GUY FAUSTIN [FR]) 3 mars 2021 (2021-03-03)
D3 ROSHDY WAEL H. ET AL: "EGYVIR: An immunomodulatory herbal extract with potent antiviral activity against SARS-CoV-2", PLOS ONE, vol. 15, no. 11, 18 novembre 2020 (2020-11-18), page e0241739, XP055846128, DOI: 10.1371/journal.pone.0241739
D4 BHATTACHARYA RIYA ET AL: "Antiviral activity of bioactive phytocompounds against coronavirus: An update", JOURNAL OF VIROLOGICAL METHODS, ELSEVIER BV, NL, vol. 290, 23 janvier 2021 (2021-01-23), XP086520479, ISSN: 0166-0934, DOI: 10.1016/J.JVIROMET.2021.114070 [extrait ie 2021-01-23]
D5 RAJAN MARIAPPAN ET AL: "Promising Antiviral Molecules from Ayurvedic Herbs and Spices against COVID-19", CHINESE JOURNAL OF INTEGRATED MEDICINE /ZHONGGUO JIEHE YIXUE ZAZHI (ENGLISH), vol. 27, no. 4, 5 février 2021 (2021-02-05), pages 243-244, XP037390321, ISSN: 1672-0415, DOI: 10.1007/S11655-021-3331-8
D6 ZÎGOLO MARÎA ANTONELA ET AL: "Virtual screening of plant-derived compounds against SARS-CoV-2 viral proteins using computational tools", SCIENCE OF THE TOTAL ENVIRONMENT, ELSEVIER, AMSTERDAM, NL, vol. 781, 17 mars 2021 (2021-03-17), XP086574866, ISSN: 0048-9697, DOI: 10.1016/J.SCITOTENV.2021.146400 [extrait le 2021-03-17]
D7 KULKARNI SEEMA A ET AL: "Computational evaluation of major components from plant essential oils as potent inhibitors of SARS-CoV-2 spike protein", JOURNAL OF MOLECULAR STRUCTURE, ELSEVIER AMSTERDAM, NL, vol. 1221, 4 juillet 2020 (2020-07-04), XP086367096, ISSN: 0022-2860, DOI: 10.1016/J.MOLSTRUC.2020.128823 [extrait ie 2020-07-04]
D8 ASIF MUHAMMAD ET AL: "COVID-19 and therapy with essential oils having antiviral, anti-inflammatory, and immunomodulatory properties", INFLAMMOPHARMACOLOGY, vol. 28, no. 5, 2020, pages 1153-1161, XP037376410, ISSN: 0925-4692, DOI: 10.1007/S10787-020-00744-0
D9 WO 2012/130893 A1 (CENTRE NAT RECH SCIENT [FR]; PASTEUR INSTITUT [FR] ET AL.) 4 octobre 2012 (2012-10-04)
D10 CLERCQ DE E: "CURRENT LEAD NATURAL PRODUCTS FOR THE CHEMOTHERAPY OF HUMAN IMMUNODEFICIENCY VIRUS (HIV) INFECTION", MEDICINAL RESEARCH REVIEWS, WILEY SUBSCRIPTION SERVICES, INC., A WILEY COMPANY, US, vol. 20, no. 5, 1 septembre 2000 (2000-09-01), pages 323-349, XP009008036, ISSN: 0198-6325, DOI: 10.1002/1098-1128(200009)20:5 323::AID- MED13.0.CO;2-A
D11 JP H08 26980 A (TSUMURA & CO) 30 janvier 1996 (1996-01-30)

### [Problème Technique a résoudre]

Un but de la présente invention est de proposer une nouvelle composition pharmaceutique utilisable en tant que médicament et plus particulièrement utilisable dans le traitement des infections par des agents pathogènes enveloppés et les maladies qu'ils produisent, leurs complications.

Un autre but de l'invention est de proposer une nouvelle composition pharmaceutique utilisable en tant que médicament et plus particulièrement utilisable dans le traitement des maladies qui remédient à tout ou une partie des inconvénients liés aux compositions de l'art antérieur précité.

Un autre but de la présente invention est de proposer une composition pharmaceutique utilisable en tant que médicament, notamment pour le traitement thérapeutique du diabète, des dyslipidémies, de l'obésité.

Un autre but de l'invention est de proposer une composition pharmaceutique qui permet d'inhiber le phénotype inflammatoire des monocytes circulants, des macrophages, des globules blancs, des cellules pancréatiques chez les patients ayant des comorbidités.

Un autre but de la présente invention est de proposer une composition pharmaceutique qui va diminuer voir inhiber l'entrée des agents pathogènes (virus, bactéries, parasites, etc.) dans leurs cellules cibles.

Un autre but de la présente invention est de proposer une composition pharmaceutique qui permet de limiter, voire inhiber l'infectiosité du SARS-CoV-2 et de ces différents variants dans les cellules, d'inhiber la réplication virale, et d'activer une réponse immunitaire compétente.

Un autre but de la présente invention est de proposer une composition pharmaceutique, notamment telle que précitée, qui présente une toxicité réduite et/ou qui est bien tolérée par les patients.

### [Brève Description de l'invention]

Pour résoudre au moins un des problèmes techniques précités, la présente invention propose une composition pharmaceutique, qui de manière caractéristique selon l'invention, comprend en tant que principe actif, une combinaison de d-limonène, de lupéol, de béta-sitostérol, la cinnamaldéhyde, et éventuellement l'épicatéchine, la curcumine et leurs mélanges selon les Revendications 1 ou 2.

### [Description détaillée]

La composition pharmaceutique peut être utilisée en tant que médicament, et notamment pour son utilisation dans le traitement préventif et curatif des infections par agent pathogène enveloppé et leurs différentes complications.

Selon un mode de réalisation particulier de la présente invention, la composition de l'invention peut comprendre, en outre, un mélange de d-limonène, de lupéol, de b-sitostérol, de cinnamaldéhyde ou un mélange de d-limonène, de lupéol, de b-sitostérol, de cinnamaldéhyde et d'Epigallocatéchine Gallate (EGCG) ou un mélange de d-limonène, de lupéol, de b-sitostérol, de cinnamaldéhyde et de curcumine ou un mélange de d-limonène, de lupéol, de b-sitostérol, de cinnamaldéhyde, d'Epigallocatéchine Gallate (EGCG) et optionellement la curcumine pour utilisation pour le traitement preventif et/ou curatif d'une infection causee par les coronaviridae (SARS-CoV, MERS-CoV, SARS-CoV-2).

A titre d'exemple, elle peut comprendre en pourcentage massique de la masse totale des ingrédients actifs, un pourcentage massique de d-limonène sensiblement égal ou supérieur à 10% et sensiblement égal ou inférieur à 55, et notamment sensiblement égal ou supérieur à 20% et sensiblement égal ou inférieur à 40%, un pourcentage de lupéol sensiblement égal ou supérieur à 15% et sensiblement égal ou inférieur à 55%, et notamment sensiblement égal ou supérieur à 30% et sensiblement égal ou inférieur à 40%, un pourcentage de beta-sitostérol sensiblement égal ou supérieur à 10% et sensiblement égal ou inférieur à 45%, et notamment sensiblement égal ou supérieur à 15% et sensiblement égal ou inférieur à 30%. un pourcentage de cinnamaldéhyde sensiblement égal ou supérieur à 15% et sensiblement égal ou inférieur à 45%, et notamment sensiblement égal ou supérieur à 20% et sensiblement égal ou inférieur à 40%, un pourcentage de Epigallocatéchine Gallate (EGCG) sensiblement égal ou supérieur à 15% et sensiblement égal ou inférieur à 40%, et notamment sensiblement égal ou supérieur à 25% et sensiblement égal ou inférieur à 35%, un pourcentage de Curcumine sensiblement égal ou supérieur à 15% et sensiblement égal ou inférieur à 40%, et notamment sensiblement égal ou supérieur à 25% et sensiblement égal ou inférieur à 35%.

La composition comprend en outre, au moins un excipient pharmaceuticalement acceptable. Cet excipient peut être solide ou liquide. Il peut être choisi, par exemple, parmi l'eau purifiée, l'alcool éthylique, le propylène glycol, la glycérine, les huiles végétales, les huiles animales, les hydrocarbures, les silicones, les sucres tels que le glucose, la cyclodextrine, le levulose, l'amidon de blé, l'amidon de mais, l'amidon de pomme de terre, la gomme xanthane, la gomme arabique, la gomme adragante, la gomme de Sterculia, la gomme Guar ou "Guaranates", les pectines, les alginates, les carraghénates, la gélose ou Agar-Agar, la gélatine, la cellulose et ses dérivés.

La composition peut être administrée par n'importe quelle voie appropriée, par exemple par la voie orale, rectale, locale (topique, par exemple), intrapéritonéale, systémique, intraveineuse, intramusculaire, sous-cutanée ou mucosale, notamment sublinguale, ou bien en utilisant un patch, ou encore sous forme encapsulée ou immobilisée sur des liposomes, des microparticules, des microcapsules, associée à des nanoparticules et analogues. On peut notamment citer, à titre d'exemples non limitatifs d'excipients appropriés pour une administration par voie orale, le talc, le lactose, l'amidon et ses dérivés, la cellulose et ses dérivés, la cyclodextrine, la piperine, les polyéthylène glycols, les polymères d'acide acrylique, la gélatine, le stéarate de magnésium, des matières grasses animales, végétales ou synthétiques, les dérivés de la paraffine, les glycols, les stabilisants, les conservateurs, les antioxydants, les agents mouillants, les antiagglomérants, les dispersants, les émulsionnants, les agents modifiants du goût, les agents de pénétrations, de solubilisation. Les techniques de formulation et d'administration des médicaments et compositions pharmaceutiques sont bien connues dans la technique ici considérée, l'Homme du Métier pouvant notamment se référer à l'ouvrage Remington's Pharmaceutical Sciences, dernière édition.

La composition peut être avantageusement administrée par voie orale, par injection en intraveineuse.

Avantageusement, la composition est adaptée pour être administrée par voie orale ou intraveineuse à une dose égale ou supérieure à 40 mg/kg/24h et égale ou inférieure à 200 mg/kg/24h en une ou plusieurs prises à un mammifère présentant un tel besoin.

A titre d'exemples, la composition peut être utilisée dans le traitement préventif et/ou curatif d'une infection causée par un agent pathogène enveloppé.

La composition peut avantageusement être utilisée dans le traitement de la maladie inflammatoire chronique et/ou résultant d'un déséquilibre glucidique et/ou d'un déséquilibre lipidique et/ou d'un dysfonctionnement du transporteur cellulaire du cholestérol et/ou d'un syndrome respiratoire aigu sévère et/ou d'une hyperactivation immunitaire systémique, notamment chez les patients infectés par un agent pathogène enveloppé et présentant des comorbidités.

Dans le cas d'une infection par un agent pathogène à enveloppe bicouche lipidique, par exemple le SARS-Cov-2, le Demandeur a mis en évidence que la composition selon l'invention donnait de bons résultats au moins in vitro et ont montré une faible cytotoxicité.

Le mode d'action de la composition n'est pas totalement élucidé. Il est plus que probable qu'elle agisse simultanément sur différents mécanismes responsables des maladies citées dans l'Art Antérieur. Elle exercerait son action par un effet régulateur synergique sur les récepteurs nucléaires, notamment les PPARs, les LXRs et les RXRs, permettant ainsi l'efflux du cholestérol cellulaire et membranaire, l'inhibition de la surexpression de certains sphingolipides, notamment les glycosphingolipides, de certaines protéines membranaires, notamment l'ACE2 l'inhibition de la production des cytokines pro-inflammatoires. Elle exercerait aussi son action par un effet immunostimulant.

Outre, la composition déstructure, restructure la composition lipidique des cellules, et notamment des radeaux lipidiques membranaires, cibles des agents pathogènes enveloppés, et empêcherait de ce fait la stabilisation de ces micro-domaines, la mise en place d'un complexe de fusion, la formation de synapse, l'endocytose, et donc à terme la pénétration de ces agents pathogènes enveloppés dans les cellules.

De plus, la modification de la composition lipidique de ces micro-domaines membranaires provoque un changement de conformation, une altération de l'activité fonctionnelle ou dysfonctionnelle des protéines qui s'y trouvent, notamment les récepteurs d'entrée du virus, les récepteurs couplés aux protéines G, et in fine une altération des voies de signalisation cellulaire impliquées dans de nombreux processus physiopathologiques, notamment dans les infections causées par des agents pathogènes, le cancer, l'obésité, les maladies métaboliques, les maladies auto-immunes et les maladies neurodégénératives.

Cette désorganisation lipidique pourrait également se faire dans l'enveloppe des agents pathogènes, et modifierait alors la conformation des protéines d'information impliquées dans le processus d'infection, leur liaison à leurs cellules cibles. Ce mécanisme s'appliquerait aux virus, bactéries ayant des propriétés biophysiques et biochimiques similaires à leurs cellules cibles, particulièrement au niveau de l'enveloppe bicouche lipidique.

La composition peut avantageusement être utilisée pour diminuer ou inhiber la résistance d'agents pathogènes et leurs variants aux médicaments, le phénotype inflammatoires des cellules, le stress oxydatif, la senescence des cellules immunitaire, et pour augmenter une réponse immunitaire innée et adaptative.

La composition selon l'invention peut être utilisée dans le traitement des infections causées par des agents pathogènes enveloppés et leurs différents variants, notamment les coronaviridae (SARS-CoV, MERS-CoV, SARS-CoV-2), les herpesviridae (Herpes simplex, Varicelle-zona, EBV, HHV-6 à 7, Herpes B du singe), les retrovirus (les lentivirus dont le VIH-1 et VIH-2, les oncovirus, et les spumavirus), les myxovirus, les paramyxoviridae (para-influenza, oreillons, rougeole, Virus Respiratoire Syncytial), virus de la rage, virus de lassa, hantavirus, virus Marburg, virus de la rubéole et dans le traitement des cancers.

Parmi ces cancers, on peut citer le sarcome de Kaposi, le lymphome de burkitt, le lymphome immunoblastique, le lymphome cérébral, primitif, les lymphomes malins non hodgkiniens (LMNH), le cancer du col de l'utérus, le cancer de la bouche, le cancer de l'estomac, le cancer du côlon, en particulier le cancer invasif du côlon ou colorectal, le cancer du rectum, le cancer anal, le cancer du foie, le carcinome hépatocellulaire, le cancer de la vésicule biliaire, le cancer du pancréas, le cancer du poumon, en particulier l'adénocarcinome du poumon, la leucémie sous la forme chronique ou aigüe, le myélome multiple, le lymphome de Hodgkin, les tumeurs de l'encéphale et d'autres à localisation au niveau du système nerveux, le cancer de la vessie, le cancer de l'ovaire, le cancer de l'utérus, le cancer du testicule, le cancer du rein, le cancer de la prostate et le cancer du sein, notamment ceux associés au tissus adipeux, les tumeurs osseuses.

La présente invention concerne également une préparation pharmaceutique qui comprend la composition selon l'invention, et, en outre, en mélange ou conditionné séparément au moins un agent anti-inflammatoire et/ou un agent antidiabétique et/ou un agent hypolipémiant et/ou un agent anti-infectieux et/ou un agent anticancéreux pour utilisation dans le traitement thérapeutique des infections causées par des agents pathogènes et leurs complications, de manière simultanée, séquencée ou espacée dans le temps.

A titre d'exemple, l'agent antidiabétique peut être choisi parmi les biguanides, les sulfamides hypoglycémiants et les glinides, les inhibiteurs de l'alpha-glucosidase, les incrétines dont le GLP-1, l'insuline, l'agent hypolipémiant peut être choisi parmi les statines, les fibrates, l'Ezétimibe, l'acide nicotinique, la cholestyramine, l'agent antiviral peut être choisi parmi les inhibiteurs nucléosidiques ou non nucléosidiques de la transcriptase inverse, les inhibiteurs de protéase, les inhibiteurs de fusion et les inhibiteurs d'intégrase, l'agent anticancéreux peut être choisi parmi les anti-métabolites (méthotrexate, capécitabine, 5-fluorouracile), les agents alkylants (cisplatine, mitomycine c, busulfan) et les apparentés (melphalan, chloraminophène, cyclophosphamide), les molécules ayant une action sur le fuseau mitotique (vinlastine, vincristine, doxétaxel), les inhibiteurs de la tyrosine kinase (afatinib, erlotinib, sunitinib), les inhibiteurs de la thréonine kinase (vermurafenib, everolimus, temsirolimus), les agents agissant sur la topo-isomérase (daunorubicine, doxorubicine, étoposide), les inhibiteurs du protéasome, les inhibiteurs de la DNA méthyltransférase, les inhibiteurs de l'histone déacétylase, les immunomodulateurs (les interférons, les corticoïdes, le talimogène), les anticorps monoclonaux (cetuximab, gemtuzumab, trastuzumab, bevacizumab, rituxumab), certains virus génétiquement modifiés qui ciblent préférentiellement les cellules cancéreuses, le gluthation, la vitamine C, le calcium folinate et leurs melanges, et notamment le mélange de deux desdits agents anticancéreux, les agents radioactifs utilisables en curiethérapie et/ou les métabolites actifs injectables ou ingérables.

La présente invention concerne également une préparation pharmaceutique qui comprend en combinaison le d-limonène, le lupéol, le béta-sitostérol, le cinnamaldéhyde et éventuellement l'épicatéchine, la curcumine et leurs mélanges.

La présente invention concerne également un complément alimentaire qui comprend en combinaison le d-limonène et/ou le lupéol et/ou la beta-sitostérol et/ou le cinnamaldéhyde et/ou et/ou l'épicatéchine et/ou la curcumine.

### [Définitions]

Les termes « traitements thérapeutiques » font référence au traitement curatif et au traitement prophylactique ; au sens de la présente invention, un traitement thérapeutique permet de restaurer au moins partiellement, de corriger au moins partiellement ou de modifier au moins partiellement des fonctions physiologiques en exerçant une action pharmacologique, immunologique ou métabolique.

Le terme « patient » fait référence à un mammifère animal ou humain. La composition selon l'invention peut également être utilisée en médecine vétérinaire.

Les termes « patients atteints de diabète » font référence aux patients atteints de diabète de type 1, les patients atteints de diabète de type 2, les patientes atteintes de diabète gestationnel, les patients atteints de diabète insipide et les patients atteints de diabète rénal.

Le terme « dyslipidémie » fait référence aux hyperlipidémies et aux hypolipidémies déterminées en fonction des critères en vigueur.

Le terme « inflammation » fait référence à un ensemble de réactions générées par l'organisme en réponse à une agression subie. Celle-ci peut être d'origine extérieure comme une blessure, une infection, un traumatisme, ou provenant de l'intérieur de l'organisme lui-même comme dans les pathologies auto-immunes.

Au sens de la présente invention, un « agent anti-cancéreux » est un élément qui présente au moins in vitro une action contre les cellules cancéreuses, indépendamment de son mécanisme d'action. Par « action » on entend, au sens de la présente invention la destruction ou la modification au moins partielle des cellules cancéreuses qui permet notamment de limiter la prolifération des cellules cancéreuses et/ou leur propagation.

Le terme « infection » désigne l'invasion d'un organisme vivant par des germes, plus précisément des micro-organismes pathogènes comme une bactérie ou un virus nécessitant un hôte, le plus souvent une cellule dont il utilise les constituants pour se multiplier.

Au sens de la présente invention, un « complément alimentaire » est une denrée alimentaire dont le but est de compléter le régime alimentaire normal et qui constitue une source concentrée de nutriments ou d'autres substances ayant un effet nutritionnel ou physiologique seuls ou combinés.

S'agissant l'agent anti-inflammatoire et/ou agent antidiabétique et/ou agent hypolipémiant et/ou agent anti-infectieux et/ou agent anticancéreux cités, les termes utilisés englobent sauf indications contraires, les isomères de constitution, les stéréo-isomères de conformation, les énantiomères et les diastéréo-isomères du composé chimique considéré.

S'agissant du cinnamaldéhyde (CA) dans la composition selon l'invention, le terme englobe sauf indications contraires, ses dérivés notamment le 2-hydroxycinnamaldehyde (HCA), 2'-benzoyloxycinnalmaldéhyde (BCA), les dimères de formation, notamment HCA-HCA, BCA-BCA, CA-CA.

S'agissant de l'épicatéchine dans la composition selon l'invention, le terme englobe sauf indications contraires, ses dérivés notamment la catéchine, la gallocatéchine (GC), le épicatéchine gallate (ECG), épigallocatéchine (EGC), épigallocatéchine gallate (EGCG).

S'agissant de la curcumine dans la composition selon l'invention, le terme englobe sauf indications contraires, ses différents analogues synthétiques.

### [Exemples]

Le pourcentage des compositions ci-dessous est un pourcentage en masse par rapport à la masse totale des ingrédients actifs.

Composition 1a : d-limonène (50%), lupéol (20%), b sitostérol (20%) et cinnamaldéhyde (10%).

Composition 1b : d-limonène (40%), lupéol (20%), le b sitostérol (30%) et cinnamaldéhyde (10%)

Composition 2a : d-limonène (40%), lupéol (10%), b sitostérol (10%), cinnamaldéhyde (10%), EGCG (30%).

Composition 2b : d-limonène (50%), lupéol (10%), b sitostérol (10%), cinnamaldéhyde (10%), EGCG (20%).

Composition 3a : d-limonène (40%), lupéol (10%), b sitostérol (10%), cinnamaldéhyde (10%), Curcumine (30%).

Composition 3b : d-limonène (50%), lupéol (10%), b sitostérol (10%), cinnamaldéhyde (10%), Curcumine (20%).

### [Resultats experimentaux]

L'activité antivirale des compositions a été évaluée en 3 étapes sur les cellules Vero E6/Vero-81 :
Première étape : Détermination de la cytotoxité des 6 molécules et des 3 compositions.
   La cytotoxicité sera déterminée par un test MTS, basé sur la bioréduction du tétrazolium MTS dans des cellules viables et métaboliquement actives en formazan coloré. Ce dernier est quantifié en mesurant l'absorbance à 490-500 nm. Cette quantité est proportionnelle au nombre de cellules vivantes en culture par rapport aux cellules non traitées. La viabilité cellulaire est mesurée après 24h d'incubation avec des concentrations croissantes des préparations à tester.
Deuxième étape : Détermination de l'activité antiviral des compositions à forte concentration.
   Les compositions seront testées en triplicats in vitro sur des cellules Vero E6/Vero-81. La plus forte dose non toxique des préparations sera mise en contact avec des cellules Vero E6/Vero-81 infectées par le SARS-CoV-2. Après 16h d'incubation à 37°C, les cellules seront lysées et l'infection virale sera mise en évidence par la révélation de la présence de la protéine de nucléocapside du SARS-CoV-2 en western blotting. La présence de la protéine de nucléocapside sera quantifiée avec le logiciel Image J.
   Go/No go : Si certaines préparations se révèlent non inhibitrices à forte concentration, celles-ci ne seront pas incluses dans la troisième étape.
Troisième étape : Détermination de la concentration inhibitrice 50 (IC50) des préparations
   L'activité antivirale des compositions sera testée en triplicat in vitro sur des cellules Vero E6/Vero-81 infectée par le Coronavirus SARS-CoV-2. Des doses croissantes des compositions seront mises en contact avec des cellules Vero E6/Vero-81 infectées par le SARS-CoV-2. Après 16h d'incubation à 37°C, les cellules seront lysées et l'infection virale sera mise en évidence par la révélation de la présence de la protéine de nucléocapside en western blotting. La présence de la protéine de nucléocapside sera quantifiée avec le logiciel Image J. Les courbes dose-réponse seront tracées et les IC50 seront déterminées.
   Résultat : Ces compositions ont une faible cytotoxicité à des concentrations biologiques acceptables. On observe également une activité antivirale significative et positive pour les différentes combinaisons contre le SARS-CoV-2. Cette composition pharmaceutique peut à juste être considérée comme un médicament candidat contre la COVID-19.

## Revendications

1. Composition pharmaceutique, **caracterisee en ce qu'**elle comprend en tant que principe actif, une combinaison de d-limonene, de lupeol, de b-sitosterol, de cinnamaldehyde, l'epicatechine, et optionnellement la curcumine, pour le traitement preventif et/ou curatif d'une infection causee par les coronaviridae (SARS-CoV, MERS-CoV, SARS-CoV-2), pour utilisation pour le traitement préventif et/ou curatif d'une infection causee par les coronaviridae (SARS-CoV, MERS-CoV, SARS-CoV-2).

2. Composition pharmaceutique **caracterisee en ce qu'**elle comprend un melange de d-limonene, de lupeol, de b-sitosterol, de cinnamaldehyde et d'Epigallocatechine Gallate (EGCG) ou un melange de d- limonene, de lupeol, de b-sitosterol, de cinnamaldehyde, d'Epigallocatechine Gallate (EGCG) et la curcumine, pour utilisation pour le traitement préventif et/ou curatif d'une infection causee par les coronaviridae (SARS-CoV, MERS-CoV, SARS-CoV-2).

3. Composition pharmaceutique pour utilisation selon la revendication 1, **caracterisee en ce qu'**elle comprend en pourcentage massique de la masse totale des ingredients actifs, un pourcentage massique de d-limonene sensiblement egal ou supérieur à10% et sensiblement egal ou inferieur a 55, et notamment sensiblement egal ou supérieur à 20% et sensiblement egal ou inférieur à 40%, un pourcentage de lupeol sensiblement egal ou supérieur à 15% et sensiblement egal ou inférieur à 55%, et notamment sensiblement egal ou supérieur à 30% et sensiblaement egal ou inférieur à 40%, un pourcentage de beta-sitosterol sensiblement egal ou supérieur à 10% et sensiblement egal ou inférieur à 45%, et notamment sensiblement egal ou supérieur à 15% et sensiblement egal ou inférieur à30%, un pourcentage de cinnamaldehyde sensiblement egal ou supérieur à 15% et sensiblement egal ou inférieur à 45%, et notamment sensiblement egal ou supérieur à 20% et sensiblement egal ou inférieur à 40%, un pourcentage de Epigallocatechine Gallate (EGCG) sensiblement egal ou supérieur à 15% et sensiblement egal ou inférieur à 40%, et notamment sensiblement egal ou supérieur à 25% et sensiblement egal ou inférieur à 35%, un pourcentage de Curcumine sensiblement egal ou supérieur à 15% et sensiblement egal ou inférieur à 40%, et notamment sensiblement egal OU supérieur à 25% et sensiblement egal OU inférieur à 35%.

4. Composition pharmaceutique selon l'une quelconque des revendications precedentes pour utilisation clans le traitement preventif et/ou curatif d'une infection causee par les coronaviridae (SARS-CoV, MERS-CoV, SARS-CoV-2).

5. Preparation pharmaceutique **caracterisee en ce qu'**elle comprend la composition selon l'une quelconque des revendications 1 a 3 et, en outre, en melange ou conditionne separement au moins un agent anti-inflammatoire et/ou un agent antidiabetique et/ou un agent hypolipemiant et/ou un agent anti-infectieux et/ou un agent anticancereux pour utilisation dans le traitement therapeutique des infections causees par les coronaviridae (SARS-CoV, MERS-CoV, SARS-CoV-2).

## Patentansprüche

1. Pharmazeutische Komposition, **dadurch gekennzeichnet, dass** sie als Wirkstoff eine Kombination von d-Limonen, Lupeol, b-Sitosterol, Cinnamaldehyd, Epikatechin sowie optional Kurkumin enthält, zur vorbeugenden und/oder heilenden Behandlung einer durch Coronaviren verursachten Infektion (SARS-CoV, MERS-CoV, SARS-CoV-2), für eine Verwendung zur vorbeugenden und/oder heilenden Behandlung einer durch Coronaviren (SARS-CoV, MERS-CoV, SARS-CoV-2) verursachten Infektion.

2. Pharmazeutische Komposition, **dadurch gekennzeichnet, dass** sie eine Mischung von d-Limonen, Lupeol, b-Sitosterol, Cinnamaldehyd und Epigallokatechin-Gallat (EGCG) oder eine Mischung von d-Limonen, Lupeol, b-Sitosterol, Cinnamaldehyd und Epigallokatechin-Gallat (EGCG) und Kurkumin enthält, für eine Verwendung zur vorbeugenden und/oder heilenden Behandlung einer durch Coronaviren (SARS-CoV, MERS-CoV, SARS-CoV-2) verursachten Infektion.

3. Pharmazeutische Komposition gemäß Anspruch 1, **dadurch gekennzeichnet, dass** sie einen Massenprozentsatz der Gesamtmasse der Wirkstoffe enthält, einen Massenprozentsatz von d-Limonen, der deutlich gleich oder größer als 0 % ist und deutlich gleich oder geringer als 55, und insbesondere deutlich gleich oder höher als 20 % und deutlich gleich oder geringer als 40 %, einen Prozentsatz von Lupeol, der deutlich gleich oder höher ist als 15 % und deutlich gleich oder geringer als 55 %, und insbesondere deutlich gleich oder höher als 30 % und deutlich gleich oder geringer als 40 %, einen Prozentsatz von Beta-Sitosterol, der deutlich gleich oder höher ist als 10 % und deutlich gleich oder geringer als 45 %, insbesondere aber deutlich gleich oder höher als 15 % und deutlich gleich oder geringer als 30 %, einen Prozentsatz von Cinnamaldehyd, der deutlich gleich oder höher ist als 15 % und deutlich gleich oder geringer als 45 %, sowie insbesondere deutlich gleich oder höher als 20 % und deutlich gleich oder geringer als 40 %, einen Prozentsatz von Epigallokatechin-Gallat (EGCG), der deutlich gleich oder höher ist als 15 % und deutlich gleich oder geringer als 40 %, und insbesondere deutlich gleich oder höher als 25 % und deutlich gleich oder geringer als 35 %, und einen Prozentsatz von Kurkumin, der deutlich gleich oder höher ist als 15 % und deutlich gleich oder geringer als 40 %, insbesondere deutlich gleich oder höher als 25 % und deutlich gleich oder geringer als 35 %.

4. Pharmazeutische Komposition gemäß einem beliebigen der vorstehenden Ansprüche, zur Verwendung in der vorbeugenden und/oder heilenden Behandlung einer durch Coronaviren (SARS-CoV, MERS-CoV, SARS-CoV-2) verursachten Infektion.

5. Pharmazeutische Komposition, **dadurch gekennzeichnet, dass** sie die Komposition gemäß einem beliebigen der Ansprüche 1 bis 3 enthält, und darüber hinaus in der Mischung oder getrennt verpackt zumindest einen entzündungshemmenden Wirkstoff und/oder einen Wirkstoff gegen Diabetes und/oder einen Wirkstoff zur Reduzierung der Blutfette und/oder einen entzündungshemmenden Wirkstoff und/oder einen Wirkstoff gegen Krebs zur Verwendung in der therapeutischen Behandlung von Infektionen, die durch Coronaviren (SARS-CoV, MERS-CoV, SARS-CoV-2) verursacht werden.

## Claims

1. Pharmaceutical composition, **characterized in that** it contains as active ingredient a combination of d-limonene, lupeol, b-sitosterol, cinnamaldehyde, epicatechin and optionally curcumin, for the preventive and/or curative treatment of an infection caused by coronaviruses (SARS-CoV, MERS-CoV, SARS-CoV-2), for use in the preventive and/or curative treatment of an infection caused by coronaviruses (SARS-CoV, MERS-CoV, SARS-CoV-2).

2. Pharmaceutical composition, **characterized in that** it contains a mixture of d-limonene, lupeol , b-sitosterol, cinnamaldehyde and epigallocatechin gallate (EGCG) or a mixture of d-limonene, lupeol , b-sitosterol, cinnamaldehyde and epigallocatechin gallate (EGCG) and curcumin, for use in the preventive and/or curative treatment of an infection caused by coronaviruses (SARS-CoV, MERS-CoV, SARS-CoV-2).

3. Pharmaceutical composition according to claim 1, **characterized in that** it contains a mass percentage of the total mass of the active ingredients, a mass percentage of d-limonene which is significantly equal to or greater than 0% and significantly equal to or less than 55%, and in particular significantly equal to or greater than 20% and significantly equal to or less than 40%, a percentage of lupeol which is significantly equal to or greater than 15% and significantly equal to or less than 55%, and in particular significantly equal to or greater than 30% and significantly equal to or less than 40%, a percentage of beta-sitosterol which is significantly equal to or greater than 10% and significantly equal to or less than 45%, but in particular significantly equal to or greater than 15% and significantly equal to or less than 30%, a percentage of cinnamaldehyde which is significantly equal to or greater than 15% and significantly equal to or less than 45%, and in particular significantly equal to or greater than 20% and significantly equal to or less than 40%, a percentage of epigallocatechin -Gallate (EGCG) which is significantly equal to or higher than 15% and significantly equal to or lower than 40%, and in particular significantly equal to or higher than 25% and significantly equal to or lower than 35%, and a percentage of curcumin which is significantly equal to or higher than 15% and significantly equal to or lower than 40%, in particular significantly equal to or higher than 25% and significantly equal to or lower than 35%.

4. Pharmaceutical composition according to any one of the preceding claims, for use in the preventive and/or curative treatment of an infection caused by coronaviruses (SARS-CoV, MERS-CoV, SARS-CoV-2).

5. Pharmaceutical composition, **characterized in that** it contains the composition according to any one of claims 1 to 3, and furthermore in the mixture or packaged separately at least one anti-inflammatory active ingredient and/or an anti-diabetic active ingredient and/or an anti -lipid active ingredient and/or an anti-cancer active ingredient for use in the therapeutic treatment of infections caused by coronaviruses (SARS-CoV, MERS-CoV, SARSCoV-2).
